# EUROPEAN PATENT APPLICATION

(11) **EP 4 273 168 A1**
(43) Date of publication of application: **08.11.2023**
(21) Application number: 21914601.6
(22) Date of filing: 30.12.2021
(51) Int. Cl.: C07K 16/28, C07K 19/00, C12N 15/13, C12N 15/62, C12N 5/10, A61K 39/00, A61K 35/17, A61P 35/00, A61P 35/02, A61P 37/02, A61P 31/04, A61P 31/12

(54) **ANTIBODY THAT SPECIFICALLY BINDS TO BCMA AND APPLICATION THEREOF**

(30) Priority: 31.12.2020 CN 202011633936
(71) Applicant: Beijing Immunochina Pharmaceuticals Co., Ltd., Beijing 100195 (CN); Beijing Yimiaoyiliao Co., Ltd., Beijing 102629 (CN)
(72) Inventor: LU, Xinan, Beijing 100195 (CN); HE, Ting, Beijing 100195 (CN); QI, Feifei, Beijing 100195 (CN); DING, Yanping, Beijing 100195 (CN); LIU, Guanghua, Beijing 100195 (CN); HU, Xuelian, Beijing 100195 (CN); XU, Wenping, Beijing 100195 (CN)
(74) Representative: Dolphin, Kirsty Mairi
(86) International application number: PCT/CN2021/142886
(87) International publication number: WO 2022/143870

(57) **Abstract**

Disclosed is an antibody that specifically binds to BCMA. The antibody comprises a heavy chain variable domain VH, a light chain variable domain VL, and a chimeric antigen receptor that contains the sequence thereof. Further disclosed are the amino acid sequence, a vector, a host cell, and a composition of the antibody or the chimeric antigen receptor of the present invention. Also disclosed is a use of the antibody or the chimeric antigen receptor in the preparation of a drug for the prevention or treatment of diseases.

## Description

### TECHNICAL FIELD

The present invention relates to an antibody that specifically binds to BCMA and a chimeric antigen receptor constructed based on the antibody, and discloses an amino acid sequencer of the antibody, a cloning or expression vector, a host cell, a method for producing the antibody, and a use of the antibody or the chimeric antigen receptor.

### BACKGROUND ART

Multiple myeloma (MM) is a malignant tumor characterized by massive proliferation of clonal plasma cells. Although treatment options for multiple myeloma have advanced significantly in recent years, multiple myeloma remains a disease with high morbidity and mortality. Therefore, new therapeutic methods are urgently needed for the treatment of multiple myeloma.

In recent years, chimeric antigen receptor-T cell (CAR-T) immunotherapy has achieved rapid development in the treatment of malignant hematological tumors, and it is one of the most effective treatment methods for malignant tumors. Chimeric antigen receptor (CAR) is a fusion protein composed of single-chain fragment variable (scFv), hinge region, transmembrane domain, intracellular signaling domain (costimulatory domain and T-cell activation domain). The scFvs of CAR-T cells are generally derived from monoclonal antibodies, which can bypass the restriction of major histocompatibility complex (MHC) to recognize intact cell surface proteins.

A candidate target in multiple myeloma immunotherapy is B Cell Maturation Antigen (BCMA). BCMA is mainly expressed by plasma cells and some mature B cells, but not expressed in most B cells and other tissues (B-cell Maturation Antigen Is a Promising Target for Adoptive T-cell Therapy of Multiple Myeloma, clinical cancer research, 15 Apr 2013*.).* Therefore, BCMA can be used as a target antigen for CAR-T cell therapy for MM. At present, clinical trials at home and abroad have confirmed that CAR-T cell therapy has achieved good therapeutic effects in the treatment of multiple myeloma. However, most of the CAR-T cells currently used in clinical trials are mouse-derived single-chain antibody scFv, which may cause CAR-T cells to generate an anti-CAR immune response *in vivo,* resulting in a decrease in the expansion ability of CAR-T cells *in vivo,* unsustainable CAR-T *in vivo* and disappointing clinical efficacy, etc. (*The novel anti-CD19 chimeric antigen receptors with humanized scFv (*single-chain variable fragment) trigger leukemia cell killing, Cellular Immunology, 14 Mar 2016*.;* Chimeric Antigen Receptor T-cell Therapies for Multiple Myeloma, blood, 14 Dec 2017*.).* In addition, the affinity of scFv to target antigen is also an important factor to determine whether CAR-T can effectively kill target cells. Therefore, screening out scFvs that can specifically and effectively recognize BCMA is crucial for the construction of a safe and effective CAR-T for the treatment for MM.

### SUMMARY OF THE INVENTION

The present invention discloses an isolated antibody that specifically binds to BCMA, comprising a heavy chain variable domain (hereinafter abbreviated as VH) and a light chain variable domain (hereinafter abbreviated as VL),
wherein the VH comprises: a VH-CDR1 comprising the amino acid sequence of SEQ ID NO: 1 (GYTFX₁X₂YSMN), a VH-CDR2 comprising the amino acid sequence of SEQ ID NO: 2 (RINTX₃SGX₄PX₅YADDFKG) and a VH-CDR3 comprising the amino acid sequence of SEQ ID NO: 3 (SNDYX₆YSLDX₇); wherein X₁ is selected from S, T, R; X₂ is selected from R, S, H; X₃ is selected from G, E, R; X₄ is selected from A, T, V; X₅ is selected from I, N; X₆ is selected from N, L; X₇ is selected from H, Y, F;
the VL comprises: a VL-CDR1 comprising the amino acid sequence of SEQ ID NO: 4 (RASX₈SVX₉X₁₀X₁₁GX₁₂X₁₃X₁₄X₁₅Y), a VL-CDR2 comprising the amino acid sequence of SEQ ID NO: 5 (LASNVQT) and a VL-CDR3 comprising the amino acid sequence of SEQ ID NO: 6 (X₁₆QSRX₁₇IPRT); wherein X₈ is selected from R, E; X₉ is selected from T, S; X₁₀ is selected from I, V; X₁₁ is selected from A, L; X₁₂ is selected from S, N; X₁₃ is selected from P, H; X₁₄ is selected from I, L; X₁₅ is selected from I, V; X₁₆ is selected from L, F; X₁₇ is selected from T, S.

In a specific embodiment, the VH of the antibody comprises: a VH-CDR1 as set forth in the amino acid sequence of SEQ ID NO: 1, a VH-CDR2 as set forth in the amino acid sequence of SEQ ID NO: 2, and an VH-CDR3 of the amino acid sequence as set forth in SEQ ID NO: 3;
the VL of the antibody comprising: a VL-CDR1 as set forth in the amino acid sequence of SEQ ID NO: 4, a VL-CDR2 as set forth in the amino acid sequence of SEQ ID NO: 5, and a VL-CDR3 as set forth in the amino acid sequence of SEQ ID NO: 6.

The present invention discloses an isolated antibody that specifically binds to BCMA, comprising a VH and a VL, the VH comprises VH-CDR1, VH-CDR2 and VH-CDR3, the VL comprises VL-CDR1, VL-CDR2 and VL-CDR3; wherein the VH-CDR1 comprises an amino acid sequence selected from the group consisting of SEQ ID NOs: 7, 8 and 9; the VH-CDR2 comprises an amino acid sequence selected from the group consisting of SEQ ID NOs: 10, 11, 12 and 13; the VH-CDR3 comprises an amino acid sequence selected from the group consisting of SEQ ID NOs: 14, 15 and 16; the VL-CDR1 comprises an amino acid sequence selected from the group consisting of SEQ ID NOs: 17, 18 and 19; the VL-CDR2 comprises the amino acid sequence of SEQ ID NO: 20; the VL-CDR3 comprises the amino acid sequence of SEQ ID NO: 21 or SEQ ID NO: 22.

In a specific embodiment, the VH-CDR1 is selected from the amino acid sequences in the group consisting of SEQ ID NOs: 7, 8 and 9; the VH-CDR2 is selected from the amino acid sequences in the group consisting of SEQ ID NOs: 10, 11, 12 and 13; the VH-CDR3 is selected from the amino acid sequences in the group consisting of SEQ ID NOs: 14, 15 and 16; the VL-CDR1 is selected from the amino acid sequences in the group consisting of SEQ ID NOs: 17, 18 and 19; the VL-CDR2 comprises the amino acid sequence of SEQ ID NO: 20; the VL-CDR3 comprises the amino acid sequence of SEQ ID NO: 21 or SEQ ID NO: 22.

In a specific embodiment, the antibody comprises:
1) a VH comprising a VH-CDR1 as set forth in SEQ ID NO: 7, a VH-CDR2 as set forth in SEQ ID NO: 10 and a VH-CDR3 as set forth in SEQ ID NO: 14; and a VL comprising a VL-CDR1 as set forth in SEQ ID NO: 17, a VL-CDR2 as set forth in SEQ ID NO: 20 and a VL-CDR3 as set forth in SEQ ID NO: 21;
2) a VH comprising a VH-CDR1 as set forth in SEQ ID NO: 8, a VH-CDR2 as set forth in SEQ ID NO: 11 and a VH-CDR3 as set forth in SEQ ID NO: 15; and a VL comprising a VL-CDR1 as set forth in SEQ ID NO: 18, a VL-CDR2 as set forth in SEQ ID NO: 20 and a VL-CDR3 as set forth in SEQ ID NO: 21;
3) a VH comprising a VH-CDR1 as set forth in SEQ ID NO: 8, a VH-CDR2 as set forth in SEQ ID NO: 12 and a VH-CDR3 as set forth in SEQ ID NO: 14; and a VL comprising a VL-CDR1 as set forth in SEQ ID NO: 17, a VL-CDR2 as set forth in SEQ ID NO: 20 and a VL-CDR3 as set forth in SEQ ID NO: 21;
4) a VH comprising a VH-CDR1 as set forth in SEQ ID NO: 7, a VH-CDR2 as set forth in SEQ ID NO: 10 and a VH-CDR3 as set forth in SEQ ID NO: 14; and a VL comprising a VL-CDR1 as set forth in SEQ ID NO: 17, a VL-CDR2 as set forth in SEQ ID NO: 20 and a VL-CDR3 as set forth in SEQ ID NO: 22; or
5) a VH comprising a VH-CDR1 as set forth in SEQ ID NO: 9, a VH-CDR2 as set forth in SEQ ID NO: 13 and a VH-CDR3 as set forth in SEQ ID NO: 16; and a VL comprising a VL-CDR1 as set forth in SEQ ID NO: 19, a VL-CDR2 as set forth in SEQ ID NO: 20 and a VL-CDR3 as set forth in SEQ ID NO: 21.

In a specific embodiment, the VH in the above antibody comprises an amino acid sequence selected from the group consisting of SEQ ID NOs: 23, 24, 25, 26, 27, 28, 29, 30, 31 and 32, or comprises an amino acid sequence with at least 85%, preferably at least 90%, more preferably at least 95%, further preferably at least 99% identity thereto.

In a specific embodiment, the VL in the above antibody comprises an amino acid sequence selected from the group consisting of SEQ ID NOs: 33, 35, 36, 37, 38, 39, 40, 41, 42 and 43, or comprises an amino acid sequence with at least 85%, preferably at least 90%, more preferably at least 95%, further preferably at least 99% identity thereto.

In a specific embodiment, the antibody comprises:
1) a VH as set forth in SEQ ID NO: 23 and a VL as set forth in SEQ ID NO: 33;
2) a VH as set forth in SEQ ID NO: 24 and a VL as set forth in SEQ ID NO: 33;
3) a VH as set forth in SEQ ID NO: 25 and a VL as set forth in SEQ ID NO: 35;
4) a VH as set forth in SEQ ID NO: 26 and a VL as set forth in SEQ ID NO: 36;
5) a VH as set forth in SEQ ID NO: 27 and a VL as set forth in SEQ ID NO: 37;
6) a VH as set forth in SEQ ID NO: 28 and a VL as set forth in SEQ ID NO: 38;
7) a VH as set forth in SEQ ID NO: 29 and a VL as set forth in SEQ ID NO: 33;
8) a VH as set forth in SEQ ID NO: 30 and a VL as set forth in SEQ ID NO: 39;
9) a VH as set forth in SEQ ID NO: 31 and a VL as set forth in SEQ ID NO: 40;
10) a VH as set forth in SEQ ID NO: 32 and a VL as set forth in SEQ ID NO: 41;
11) a VH as set forth in SEQ ID NO: 28 and a VL as set forth in SEQ ID NO: 42; or
12) a VH as set forth in SEQ ID NO: 29 and a VL as set forth in SEQ ID NO: 43.

In a specific embodiment, the above antibody comprises an amino acid sequence selected from the group consisting of SEQ ID NO: 44, 45, 46, 47, 48, 49, 50, 51, 52, 53, 54, 55 and 56, or comprises an amino acid sequence with at least 85%, preferably at least 90%, more preferably at least 95%, further preferably at least 99% identity thereto; or
the antibody consists of, or consists essentially of the above sequences.

In a specific embodiment, the antibody of the present invention is a single-chain antibody scFv with its VH and VL linked by glycine- and serine-rich linker peptide chains, and the order of the VH and the VL is interchangeable.

In a specific embodiment, the antibody of the present invention is a chimeric or humanized antibody.

In the other aspect, the present invention further discloses a chimeric antigen receptor targeting BCMA, comprising: 1) a scFv of the present invention; 2) hinge region; 3) transmembrane region; 4) costimulatory domain; and 5) CD3ζ intracellular signal transduction domain. Wherein the costimulatory domain is selected from CD27, CD28, 4-1BB, OX-40, CD30, CD40, PD-1, ICOS, LFA-1, CD-2, CD7, LIGHT, NKG2C, B7-H3, or any combination thereof; preferably, the costimulatory domain is selected from 4-1BB, the 4-1BB comprises the amino acid sequence of SEQ ID NO. 57; the CD3ζ intracellular signal transduction domain comprises the amino acid sequence of SEQ ID NO. 58.

In a specific embodiment, the hinge region and the transmembrane domain are selected from the hinge region and transmembrane domain of IgG1, IgG4, CD8α, CD28, IL-2 receptor, IL-7 receptor, IL-11 receptor, PD-1 or CD34.

The sequences of the exemplary antibodies are as set forth in Table 1 and in the sequence listing.

**Table 1 Variable Region Sequences of Exemplary Anti-BCMA scFv**

| | SEQ ID NO | amino acid sequence | Note |
|---|---|---|---|
| VH-CDR1 | 1 | GYTFX₁X₂YSMN | X₁ is selected from S, T, R; X₂ is selected from R, S, H; |
| VH-CDR2 | 2 | RINTX₃SGX₄P X₅YADDFKG | X₃ is selected from G, E, R; X₄ is selected from A, T, V; X₅ is selected from I, N; |
| VH-CDR3 | 3 | SNDYX₆YSLD X₇ | X₆ is selected from N, L; X₇ is selected from H, Y, F; |
| VL-CDR1 | 4 | RASX₈SVX₉X₁₀X₁₁GX₁₂X₁₃X₁₄X₁₅Y | X₈ is selected from R, E; X₉ is selected from T, S; X₁₀ is selected from I, V; X₁₁ is selected from A, L; X₁₂ is selected from S, N; X₁₃ is selected from P, H; X₁₄ is selected from I, L; X₁₅ is selected from I, V; |
| VL-CDR2 | 5 | LASNVQT | - |
| VL-CDR3 | 6 | X₁₆QSRX₁₇IPRT | X₁₆ is selected from L, F; X₁₇ is selected from T, S; |

The CDR sequences of the exemplary antibodies are as set forth in Table 2 and the sequence listing.

**Table 2 CDR Sequences of Exemplary Antibodies**

| Clone ID. | | SEQ ID NO | amino acid sequence |
|---|---|---|---|
| scFv-01 | VH-CDR1 | 7 | GYTFSRYSMN |
| | VH-CDR2 | 10 | RINTGSGAPIYADDFKG |
| | VH-CDR3 | 14 | SNDYNYSLDH |
| | VL-CDR1 | 17 | RASRSVTILGNPIVY |
| | VL-CDR2 | 20 | LASNVQT |
| | VL-CDR3 | 21 | LQSRTIPRT |
| | VH | 23 | QVQLVQSGPELKKPGETVKISCKASGYTFSRYSMNWVRQAPGKGLEWMGRINTG SGAPIYADDFKGRFIFSVDTSASTAYLVINNLKSEDTASYFCSNDYNYSLDHWGQGT AVTVSS |
| | VL | 33 | DIVLTQSPSSLSVSLGQRATISCRASRSVTILGNPIVYWYQQKPGQPPVLLIQLASNV QTGVPARFSGSGSGTDFTLTIDPVEEDDVAVYYCLQSRTIPRTFGGGTKLEIK |
| | scFv | 44 | QVQLVQSGPELKKPGETVKISCKASGYTFSRYSMNWVRQAPGKGLEWMGRINTG SGAPIYADDFKGRFIFSVDTSASTAYLVINNLKSEDTASYFCSNDYNYSLDHWGQGT AVTVSSGSTSGSGKPGSGEGSTKGDIVLTQSPSSLSVSLGQRATISCRASRSVTILGNP IVYWYQQKPGQPPVLLIQLASNVQTGVPARFSGSGSGTDFTLTIDPVEEDDVAVYY CLQSRTIPRTFGGGTKLEIK |
| scFv-04 | VH-CDR1 | 7 | GYTFSRYSMN |
| | VH-CDR2 | 10 | RINTGSGAPIYADDFKG |
| | VH-CDR3 | 14 | SNDYNYSLDH |
| | VL-CDR1 | 17 | RASRSVTILGNPIVY |
| | VL-CDR2 | 20 | LASNVQT |
| | VL-CDR3 | 21 | LQSRTIPRT |
| | VH | 24 | QVQLVQSGPELKKPGETVKISCKASGYTFSRYSMNWVRQAPGKGLEWMGRINTG SGAPIYADDFKGRFTFSVDTSASTAYLVINNLKSEDTASYFCSNDYNYSLDHWGQG TAVTVSS |
| | VL | 33 | DIVLTQSPSSLSVSLGQRATISCRASRSVTILGNPIVYWYQQKPGQPPVLLIQLASNV QTGVPARFSGSGSGTDFTLTIDPVEEDDVAVYYCLQSRTIPRTFGGGTKLEIK |
| | scFv | 45 | QVQLVQSGPELKKPGETVKISCKASGYTFSRYSMNWVRQAPGKGLEWMGRINTG SGAPIYADDFKGRFTFSVDTSASTAYLVINNLKSEDTASYFCSNDYNYSLDHWGQG TAVTVSSGSTSGSGKPGSGEGSTKGDIVLTQSPSSLSVSLGQRATISCRASRSVTILGN PIVYWYQQKPGQPPVLLIQLASNVQTGVPARFSGSGSGTDFTLTIDPVEEDDVAVYY CLQSRTIPRTFGGGTKLEIK |
| scFv-05 | VH-CDR1 | 7 | GYTFSRYSMN |
| | VH-CDR2 | 10 | RINTGSGAPIYADDFKG |
| | VH-CDR3 | 14 | SNDYNYSLDH |
| | VL-CDR1 | 17 | RASRSVTILGNPIVY |
| | VL-CDR2 | 20 | LASNVQT |
| | VL-CDR3 | 22 | FQSRSIPRT |
| | VH | 24 | QVQLVQSGPELKKPGETVKISCKASGYTFSRYSMNWVRQAPGKGLEWMGRINTG SGAPIYADDFKGRFTFSVDTSASTAYLVINNLKSEDTASYFCSNDYNYSLDHWGQG TAVTVSS |
| | VL | 34 | DIVLTQSPSSLSVSLGQRATISCRASRSVTILGNPIVYWYQQKPGQPPVLLIQLASNV QTGVPARFSGSGSGTDFTLTIDPVEEDDVAVYYCFQSRSIPRTFGGGTKLEIK |
| | scFv | 46 | QVQLVQSGPELKKPGETVKISCKASGYTFSRYSMNWVRQAPGKGLEWMGRINTG SGAPIYADDFKGRFTFSVDTSASTAYLVINNLKSEDTASYFCSNDYNYSLDHWGQG TAVTVSSGSTSGSGKPGSGEGSTKGDIVLTQSPSSLSVSLGQRATISCRASRSVTILGN PIVYWYQQKPGQPPVLLIQLASNVQTGVPARFSGSGSGTDFTLTIDPVEEDDVAVYY CFQSRSIPRTFGGGTKLEIK |
| scFv-06 | VH-CDR1 | 7 | GYTFSRYSMN |
| | VH-CDR2 | 10 | RINTGSGAPIYADDFKG |
| | VH-CDR3 | 14 | SNDYNYSLDH |
| | VL-CDR1 | 17 | RASRSVTILGNPIVY |
| | VL-CDR2 | 20 | LASNVQT |
| | VL-CDR3 | 21 | LQSRTIPRT |
| | VH | 25 | QVQLVQSGPEVKKPGESVKVSCKASGYTFSRYSMNWVRQAPGQGLEWMGRINT GSGAPIYADDFKGRFTFTVDESTSTAYLVLNNLRSEDTAVYFCSNDYNYSLDHWGQ GTAVTVSS |
| | VL | 35 | EIVLTQSPSTLSVSLGQRATLSCRASRSVTILGNPIVYWYQQKPGQPPVLLIQLASNV QTGIPARFSGSGSGTDFTLTIDPLEEEDVAVYYCLQSRTIPRTFGGGTKLEIK |
| | scFv | 47 | QVQLVQSGPEVKKPGESVKVSCKASGYTFSRYSMNWVRQAPGQGLEWMGRINT GSGAPIYADDFKGRFTFTVDESTSTAYLVLNNLRSEDTAVYFCSNDYNYSLDHWGQ GTAVTVSSGSTSGSGKPGSGEGSTKGEIVLTQSPSTLSVSLGQRATLSCRASRSVTIL GNPIVYWYQQKPGQPPVLLIQLASNVQTGIPARFSGSGSGTDFTLTIDPLEEEDVAV YYCLQSRTIPRTFGGGTKLEIK |
| scFv-07 | VH-CDR1 | 9 | GYTFRHYSMN |
| | VH-CDR2 | 13 | RINTESGVPIYADDFKG |
| | VH-CDR3 | 16 | SNDYLYSLDF |
| | VL-CDR1 | 19 | RASESVTILGSHLIY |
| | VL-CDR2 | 20 | LASNVQT |
| | VL-CDR3 | 21 | LQSRTIPRT |
| | VH | 26 | QVQLVQSGPEVKKPGESVKVSCKASGYTFRHYSMNWVRQAPGQGLEWMGRINT ESGVPIYADDFKGRFAFTVDESTSTAYLVLNNLRSEDTAVYFCSNDYLYSLDFWGQ GTAVTVSS |
| | VL | 36 | EIVLTQSPPTLAMSLGKRATLSCRASESVTILGSHLIYWYQQKPGQPPTLLIQLASNV QTGIPARFSGSGSRTDFTLTIDPLEEEDVAVYYCLQSRTIPRTFGGGTKLEIK |
| | scFv | 48 | QVQLVQSGPEVKKPGESVKVSCKASGYTFRHYSMNWVRQAPGQGLEWMGRINT ESGVPIYADDFKGRFAFTVDESTSTAYLVLNNLRSEDTAVYFCSNDYLYSLDFWGQ GTAVTVSSGSTSGSGKPGSGEGSTKGEIVLTQSPPTLAMSLGKRATLSCRASESVTIL GSHLIYWYQQKPGQPPTLLIQLASNVQTGIPARFSGSGSRTDFTLTIDPLEEEDVAVY YCLQSRTIPRTFGGGTKLEIK |
| scFv-08 | VH-CDR1 | 8 | GYTFTSYSMN |
| | VH-CDR2 | 11 | RINTESGAPNYADDFKG |
| | VH-CDR3 | 15 | SNDYLYSLDY |
| | VL-CDR1 | 18 | RASRSVSVAGNHIVY |
| | VL-CDR2 | 20 | LASNVQT |
| | VL-CDR3 | 21 | LQSRTIPRT |
| | VH | 27 | QVQLVQSGPEVKKPGESVKVSCKASGYTFTSYSMNWVRQVPGQGLEWMGRINT |
| | | | ESGAPNYADDFKGRFTFTVDTSTSTAYLEINNLRSEDTAVYFCSNDYLYSLDYWGQ GTAVTVSS |
| | VL | 37 | EIVLTQSPSTLSVSLGQRATLSCRASRSVSVAGNHIVYWYQQKPGQPPVLLIQLASN VQTGIPARFSGSGSGTDFTLTIDSLEPEDVAVYYCLQSRTIPRTFGGGTKLEIK |
| | scFv | 49 | QVQLVQSGPEVKKPGESVKVSCKASGYTFTSYSMNWVRQVPGQGLEWMGRINT ESGAPNYADDFKGRFTFTVDTSTSTAYLEINNLRSEDTAVYFCSNDYLYSLDYWGQ GTAVTVSSGSTSGSGKPGSGEGSTKGEIVLTQSPSTLSVSLGQRATLSCRASRSVSVA GNHIVYWYQQKPGQPPVLLIQLASNVQTGIPARFSGSGSGTDFTLTIDSLEPEDVAV YYCLQSRTIPRTFGGGTKLEIK |
| scFv-09 | VH-CDR1 | 7 | GYTFSRYSMN |
| | VH-CDR2 | 10 | RINTGSGAPIYADDFKG |
| | VH-CDR3 | 14 | SNDYNYSLDH |
| | VL-CDR1 | 17 | RASRSVTILGNPIVY |
| | VL-CDR2 | 20 | LASNVQT |
| | VL-CDR3 | 21 | LQSRTIPRT |
| | VH | 28 | QVQLVQSGPELKKPGETVKISCKASGYTFSRYSMNWVRQAPGKGLEWMGRINTG SGAPIYADDFKGRFIFSVDTSASTAYMELSSLRSEDTAVYYCSNDYNYSLDHWGQG TAVTVSS |
| | VL | 38 | EIVLTQSPATLSVSLGQRATISCRASRSVTILGNPIVYWYQQKPGQPPVLLIQLASNV QTGVPARFSGSGSGTDFTLTISSLEEDDVAVYYCLQSRTIPRTFGGGTKLEIK |
| | scFv | 50 | QVQLVQSGPELKKPGETVKISCKASGYTFSRYSMNWVRQAPGKGLEWMGRINTG SGAPIYADDFKGRFIFSVDTSASTAYMELSSLRSEDTAVYYCSNDYNYSLDHWGQG TAVTVSSGSTSGSGKPGSGEGSTKGEIVLTQSPATLSVSLGQRATISCRASRSVTILGN PIVYWYQQKPGQPPVLLIQLASNVQTGVPARFSGSGSGTDFTLTISSLEEDDVAVYY CLQSRTIPRTFGGGTKLEIK |
| scFv-10 | VH-CDR1 | 8 | GYTFTSYSMN |
| | VH-CDR2 | 12 | RINTRSGTPNYADDFKG |
| | VH-CDR3 | 14 | SNDYNYSLDH |
| | VL-CDR1 | 17 | RASRSVTILGNPIVY |
| | VL-CDR2 | 20 | LASNVQT |
| | VL-CDR3 | 21 | LQSRTIPRT |
| | VH | 29 | QVQLVQSGAEVKKPGASVKVSCKASGYTFTSYSMNWVRQAPGQGLEWMGRINT RSGTPNYADDFKGRVTITADESVSTAYMELSNLKSEDTASYFCSNDYNYSLDHWG QGTAVTVSS |
| | VL | 33 | DIVLTQSPSSLSVSLGQRATISCRASRSVTILGNPIVYWYQQKPGQPPVLLIQLASNV QTGVPARFSGSGSGTDFTLTIDPVEEDDVAVYYCLQSRTIPRTFGGGTKLEIK |
| | scFv | 51 | QVQLVQSGAEVKKPGASVKVSCKASGYTFTSYSMNWVRQAPGQGLEWMGRINT RSGTPNYADDFKGRVTITADESVSTAYMELSNLKSEDTASYFCSNDYNYSLDHWG QGTAVTVSSGGSTSGSGKPGSGEGSTKGDIVLTQSPSSLSVSLGQRATISCRASRSVTIL GNPIVYWYQQKPGQPPVLLIQLASNVQTGVPARFSGSGSGTDFTLTIDPVEEDDVAV YYCLQSRTIPRTFGGGTKLEIK |
| scFv-11 | VH-CDR1 | 7 | GYTFSRYSMN |
| | VH-CDR2 | 10 | RINTGSGAPIYADDFKG |
| | VH-CDR3 | 14 | SNDYNYSLDH |
| | VL-CDR1 | 17 | RASRSVTILGNPIVY |
| | VL-CDR2 | 20 | LASNVQT |
| | VL-CDR3 | 21 | LQSRTIPRT |
| | VH | 30 | QVQLVQSGPEVKKPGASVKVSCKASGYTFSRYSMNWVRQAPGQGLEWMGRINT GSGAPIYADDFKGRVTMTRDESTSTAYLVLNNLRSEDTAVYFCSNDYNYSLDHWG QGTAVTVSS |
| | VL | 39 | EIVMTQSPPTLSVSLGQRATLSCRASRSVTILGNPIVYWYQQKAPKPPVLLIQLASN VQTGIPARFSGSGSGTDFTLTIDPLEEEDVAVYYCLQSRTIPRTFGGGTKLEIK |
| | scFv | 52 | QVQLVQSGPEVKKPGASVKVSCKASGYTFSRYSMNWVRQAPGQGLEWMGRINT GSGAPIYADDFKGRVTMTRDESTSTAYLVLNNLRSEDTAVYFCSNDYNYSLDHWG QGTAVTVSSGSTSGSGKPGSGEGSTKGEIVMTQSPPTLSVSLGQRATLSCRASRSVTI LGNPIVYWYQQKAPKPPVLLIQLASNVQTGIPARFSGSGSGTDFTLTIDPLEEEDVAV YYCLQSRTIPRTFGGGTKLEIK |
| scFv-12 | VH-CDR1 | 9 | GYTFRHYSMN |
| | VH-CDR2 | 13 | RINTESGVPIYADDFKG |
| | VH-CDR3 | 16 | SNDYLYSLDF |
| | VL-CDR1 | 19 | RASESVTILGSHLIY |
| | VL-CDR2 | 20 | LASNVQT |
| | VL-CDR3 | 21 | LQSRTIPRT |
| | VH | 31 | EVQLVESGGGLVQPGESVKVSCKASGYTFRHYSMNWVRQAPGQGLEWMGRINT ESGVPIYADDFKGRFTISRDESTSTAYLVLNNLRSEDTAVYFCSNDYLYSLDFWGQG |
| | | | TAVTVSS |
| | VL | 40 | EIVLTQSPPTLAMSLGKRATLSCRASESVTILGSHLIYWYQQKPGQPPTLLIQLASNV QTGIPARFSGSGSRTDFTLTIDPLEEEDVAVYYCLQSRTIPRTFGGGTKVEIK |
| | scFv | 53 | EVQLVESGGGLVQPGESVKVSCKASGYTFRHYSMNWVRQAPGQGLEWMGRINT ESGVPIYADDFKGRFTISRDESTSTAYLVLNNLRSEDTAVYFCSNDYLYSLDFWGQG TAVTVSSGSTSGSGKPGSGEGSTKGEIVLTQSPPTLAMSLGKRATLSCRASESVTILG SHLIYWYQQKPGQPPTLLIQLASNVQTGIPARFSGSGSRTDFTLTIDPLEEEDVAVYY CLQSRTIPRTFGGGTKVEIK |
| scFv-13 | VH-CDR1 | 8 | GYTFTSYSMN |
| | VH-CDR2 | 11 | RINTESGAPNYADDFKG |
| | VH-CDR3 | 15 | SNDYLYSLDY |
| | VL-CDR1 | 18 | RASRSVSVAGNHIVY |
| | VL-CDR2 | 20 | LASNVQT |
| | VL-CDR3 | 21 | LQSRTIPRT |
| | VH | 32 | EVQLVESGGGVKKPGESVKVSCKASGYTFTSYSMNWVRQVPGQGLEWMGRINT ESGAPNYADDFKGRFTISRDTSTNSLYLEINNLRSEDTAVYFCSNDYLYSLDYWGQ GTAVTVSS |
| | VL | 41 | EIVLTQSPSTLSVSLGQRATLSCRASRSVSVAGNHIVYWYQQKPGQPPVLLIQLASN VQTGIPARFSGSGSGTDFTLTISSLEPEDVAVYYCLQSRTIPRTFGGGTKLEIK |
| | scFv | 54 | EVQLVESGGGVKKPGESVKVSCKASGYTFTSYSMNWVRQVPGQGLEWMGRINT ESGAPNYADDFKGRFTISRDTSTNSLYLEINNLRSEDTAVYFCSNDYLYSLDYWGQ GTAVTVSSGSTSGSGKPGSGEGSTKGEIVLTQSPSTLSVSLGQRATLSCRASRSVSVA GNHIVYWYQQKPGQPPVLLIQLASNVQTGIPARFSGSGSGTDFTLTISSLEPEDVAVY YCLQSRTIPRTFGGGTKLEIK |
| scFv-14 | VH-CDR1 | 7 | GYTFSRYSMN |
| | VH-CDR2 | 10 | RINTGSGAPIYADDFKG |
| | VH-CDR3 | 14 | SNDYNYSLDH |
| | VL-CDR1 | 17 | RASRSVTILGNPIVY |
| | VL-CDR2 | 20 | LASNVQT |
| | VL-CDR3 | 21 | LQSRTIPRT |
| | VH | 28 | QVQLVQSGPELKKPGETVKISCKASGYTFSRYSMNWVRQAPGKGLEWMGRINTG SGAPIYADDFKGRFIFSVDTSASTAYMELSSLRSEDTAVYYCSNDYNYSLDHWGQG TAVTVSS |
| | VL | 42 | EIVLTQSPATLSVSLGQRATISCRASRSVTILGNPIVYWYQQKPGQPPVLLIQLASNV QTGVPARFSGSGSGTDFTLTISSLQSEDFAVYYCLQSRTIPRTFGGGTKLEIK |
| | scFv | 55 | QVQLVQSGPELKKPGETVKISCKASGYTFSRYSMNWVRQAPGKGLEWMGRINTG SGAPIYADDFKGRFIFSVDTSASTAYMELSSLRSEDTAVYYCSNDYNYSLDHWGQG TAVTVSSGSTSGSGKPGSGEGSTKGEIVLTQSPATLSVSLGQRATISCRASRSVTILGN PIVYWYQQKPGQPPVLLIQLASNVQTGVPARFSGSGSGTDFTLTISSLQSEDFAVYY CLQSRTIPRTFGGGTKLEIK |
| scFv-15 | VH-CDR1 | 8 | GYTFTSYSMN |
| | VH-CDR2 | 12 | RINTRSGTPNYADDFKG |
| | VH-CDR3 | 14 | SNDYNYSLDH |
| | VL-CDR1 | 17 | RASRSVTILGNPIVY |
| | VL-CDR2 | 20 | LASNVQT |
| | VL-CDR3 | 21 | LQSRTIPRT |
| | VH | 29 | QVQLVQSGAEVKKPGASVKVSCKASGYTFTSYSMNWVRQAPGQGLEWMGRINT RSGTPNYADDFKGRVTITADESVSTAYMELSNLKSEDTASYFCSNDYNYSLDHWG QGTAVTVSS |
| | VL | 43 | DIVLTQSPSSLSVSLGQRATISCRASRSVTILGNPIVYWYQQKPGQPPVLLIQLASNV QTGVPARFSGSGSGTDFTLTISSLQSEDFAVYYCLQSRTIPRTFGGGTKLEIK |
| | scFv | 56 | QVQLVQSGAEVKKPGASVKVSCKASGYTFTSYSMNWVRQAPGQGLEWMGRINT RSGTPNYADDFKGRVTITADESVSTAYMELSNLKSEDTASYFCSNDYNYSLDHWG QGTAVTVSSGSTSGSGKPGSGEGSTKGDIVLTQSPSSLSVSLGQRATISCRASRSVTIL GNPIVYWYQQKPGQPPVLLIQLASNVQTGVPARFSGSGSGTDFTLTISSLQSEDFAV YYCLQSRTIPRTFGGGTKLEIK |

The present invention discloses an isolated nucleic acid molecule encoding the aforementioned antibody or chimeric antigen receptor.

The present invention also discloses a cloning or expression vector comprising the aforementioned nucleic acid molecule. Wherein the vector is selected from one or more of DNA, RNA, plasmid, lentiviral vector, adenoviral vector and retroviral vector.

The present invention also discloses a host cell comprising the aforementioned nucleic acid molecule or the aforementioned vector. Wherein the host cell is a T lymphocyte, a B lymphocyte, a natural killer cell, a dendritic cell, a macrophage, a cytotoxic T cell, a tumorinfiltrating T cell or a Treg (regulatory T cell).

The present invention also discloses a composition or kit comprising at least one of the aforementioned antibody, the aforementioned chimeric antigen receptor, the aforementioned nucleic acid molecule, the aforementioned vector, the aforementioned host cell, and pharmaceutically acceptable excipient(s), diluent(s) or carrier(s).

The present invention also discloses a use of the aforementioned antibody, the aforementioned chimeric antigen receptor, the aforementioned nucleic acid molecule, the aforementioned vector, the aforementioned host cell, or the aforementioned composition in the preparation of a medicament for the prevention or treatment of B-cell associated neoplastic diseases, autoimmune diseases, infectious diseases caused by viruses or bacteria.

The present invention discloses a method of treating a disease, wherein an effective amount of the aforementioned antibody, the aforementioned chimeric antigen receptor, the aforementioned nucleic acid molecule, the aforementioned vector, the aforementioned host cell, or the aforementioned composition is administered to a subject in need of treatment, wherein the subject suffers from at least one of B-cell associated neoplastic diseases, autoimmune diseases, infectious diseases caused by viruses or bacteria.

In one embodiment, the B-cell associated neoplastic disease is at least one selected from: acute leukemias such as acute lymphoblastic leukemia, acute myelocytic leukemia, acute myelogenous leukemia and myeloblastic leukemia, promyelocytic leukaemia, myelomonocytic leukaemia, monocytic leukaemia and erythroleukemia; chronic leukemias such as chronic myelocytic (granulocytic) leukemia, chronic myelogenous leukemia, and chronic lymphocytic leukemia; polycythemia vera, lymphoma, mantle cell lymphoma, diffuse large B-cell lymphoma, Hodgkin's disease, non-Hodgkin's lymphoma, multiple myeloma, Waldenstrom's macroglobulinemia, heavy chain disease, myelodysplastic syndrome, hairy cell leukemia, and myelodysplasia.

### BRIEF DESCRIPTION OF THE DRAWINGS

Figure 1 shows a schematic diagram of a CAR molecule.
Figure 2 shows a schematic diagram of a plasmid vector. The plasmid used for the preparation of lentivirus is pLenti plasmid, the upstream promoter of the target gene is EF-1α, and the downstream of the promoter are signal peptide, linker region and CAR structural sequence in order.
Figure 3 shows the transduction efficiency in T cells of the lentivirus with a CAR bearing a scFv that specifically recognizes BCMA.
Figure 4 shows the flow assay plots of BCMA expression on the surface of NCI H929 cells and K562 cells.
Figure 5 shows the graph of the killing effect of CAR-T containing scFv-01 (01-CAR-T) on target cells, wherein 01-CAR-T indicates CAR-T cells containing scFv-01, C-CAR-T is a positive control, and T cell treatment group is a negative control.
Figure 6 shows the graph of the killing effect of CAR-T containing scFv-04 (04-CAR-T) on target cells.
Figure 7 shows the graph of the killing effect of CAR-T containing scFv-05 (05-CAR-T) on target cells.
Figure 8 shows the graph of the killing effect of CAR-T containing scFv-06 (06-CAR-T) on target cells.
Figure 9 shows the graph of the killing effect of CAR-T containing scFv-07 (07-CAR-T) on target cells.
Figure 10 shows the graph of the killing effect of CAR-T containing scFv-08 (08-CAR-T) on target cells.
Figure 11 shows the graph of the killing effect of CAR-T containing scFv-09 (09-CAR-T) on target cells.
Figure 12 shows the graph of the killing effect of CAR-T containing scFv-10 (10-CAR-T) on target cells.
Figure 13 shows the graph of the killing effect of CAR-T containing scFv-11 (11-CAR-T) on target cells.
Figure 14 shows the graph of the killing effect of CAR-T containing scFv-12 (12-CAR-T) on target cells.
Figure 15 shows the graph of the killing effect of CAR-T containing scFv-13 (13-CAR-T) on target cells.
Figure 16 shows the graph of the killing effect of CAR-T containing scFv-14 (14-CAR-T) on target cells.
Figure 17 shows the graph of the killing effect of CAR-T containing scFv-15 (15-CAR-T) on target cells.
Figure 18 shows a normalized graph of the killing effect of CAR-T in the present invention on target cells.
Figure 19 shows a graph of cytokine expression of CAR-T containing scFv-01 (01-CAR-T) upon target cell stimulation.

### DETAILED DESCRIPTION

The present invention will be further described below through specific embodiments. Unless otherwise defined, terms used herein have the same meaning as commonly understood by those skilled in the art.

The term "antigen", as used herein, refers to a molecule that elicits an immune response, which may involve antibody production, or activation of specific immunocompetent cells. Those skilled in the art will understand that any macromolecule, including all proteins or peptides, can be used as an antigen. Antigens can be derived from recombinant or genomic DNA. Those skilled in the art will understand any DNA that includes a nucleotide sequence or part of a nucleotide sequence encoding a protein that elicits an immune response, encoding the term "antigen" as used herein. Furthermore, those skilled in the art will understand that the antigen need not be encoded solely by the full-length nucleotide sequence of a gene. It is readily apparent that the present disclosure includes, but is not limited to, a use of more than one partial nucleotide sequences of gene, and that these nucleotide sequences are arranged in different combinations to elicit a desired immune response. Furthermore, those skilled in the art will understand that the antigen need not be encoded by a "gene" at all, the antigen may be generated, synthesized or derived from a biological sample. Such biological samples may include, but are not limited to, tissue samples, tumor samples, cells, or biological fluids.

The term "antibody", as used herein, refers to an immunoglobulin molecule that specifically binds to an antigen. Antibodies can be intact immunoglobulins derived from natural sources or from recombinant sources, and can be immunoreactive portions of intact immunoglobulins. Antibodies are typically tetramers of immunoglobulin molecules. The antibodies in the present invention may exist in a variety of forms including, for example, polyclonal, monoclonal, monospecific, multispecific, nonspecific, humanized, single-chain, chimeric, synthetic, recombinant, hybrid, mutated, and grafted antibody; the forms of the antibodies in the present invention also includs full-length antibodies, antibody fragment, for example, Fab, Fab', F(ab')₂, Fv, scFv, di-scFv, tri-scFv, Fd and other antibody fragments that retain antigen-binding function; they may also be dimeric structures (Diabody) or trimeric structures (Triabody). Typically, the fragment should include an antigen-binding fragment, which typically comprises a VL and a VH, however, it does not necessarily have to comprise both. For example, so-called Fd antibody fragments consist only of VH and CH1 domains, but still retain some of the antigen-binding functions of the intact antibody. The term "antibody" as an immunoglobulin or fragment or derivative thereof, includes any polypeptide comprising an antigen binding site, whether produced *in vitro* or *in vivo.* The VH or VL regions can be further subdivided into hypervariable regions called complementarity determining regions (CDRs), and more conserved regions called framework regions (FWRs) that is interspersed. The variable regions of the heavy and light chains comprise binding domains that interact with antigen. The CDRs in heavy chain are abbreviated as VH-CDRs, e.g., VH-CDR1, VH-CDR2, VH-CDR3, and the CDRs in light chain are abbreviated as VL-CDRs, e.g., VL-CDR1, VL-CDR2, VL-CDR3. The CDRs of the antibodies and antigen-binding fragments disclosed in the present invention are defined or identified by Kabat numbering.

The terms "single-chain variable region fragment", "single-chain antibody" or "scfv", as used herein, refer to an antibody formed by recombinant DNA techniques in which the heavey chain variable region and light chain variable region of immunoglobulin are linked by amino acid peptides segments (linker). Various methods for generating single-chain antibodies are known, including those described in US Pat. No. 4,694,778; Bird (1988) Science 242:423-442; Huston et al (1988) Proc. Natl. Acad. Sci. USA 85:5879-5883; Ward et al (1989) Nature 334:54454; Skerra et al (1988) Science 242:1038-1041. The variable regions of heavy chain and light chain comprise binding domains that interact with antigen. The CDRs in heavy chain are abbreviated as VH-CDRs, e.g., VH-CDR1, VH-CDR2, VH-CDR3, and the CDRs in light chain are abbreviated as VL-CDRs, e.g., VL-CDR1, VL-CDR2, VL-CDR3. The CDRs of the antibodies and antigen-binding fragments disclosed in the present invention are defined or identified by Kabat numbering.

The term "chimeric", as used in the present disclosure, refers to an antibody or antibody polypeptide in which a portion of the heavy chain is derived from one species and the other portion of the heavy chain is derived from other species. In an illustrative example, a chimeric antibody may comprise a constant region derived from human and a variable region derived from non-human animal such as camelids. In certain embodiments, the non-human animal is a mammal, such as a camelid, mouse, rat, rabbit, goat, sheep, guinea pig, or hamster.

The term "humanized", as used in the present disclosure, refers to an antibody or antibody polypeptide comprising CDRs derived from non-human animal, FWR regions derived from human, and when applicable, constant regions derived from human.

"BCMA", as used in the present disclosure, can be derived from human, and exemplary sequences of human BCMA include human BCMA protein (Genbank accession No.: BAB60895.1).

The term "BCMA", as used the present invention, is intended to encompass any form of BCMA, for example, 1) natural unprocessed BCMA molecules, "full length" BCMA chains or naturally generated BCMA variants, including, e.g., splice variants or allelic variants; 2) any form of BCMA produced after intracellular processing; or 3) full-length, fragments (for example, truncated forms, extracellular/transmembrane domains) of BCMA subunits , or modified forms thereof (for example, mutant forms, glycosylation/pegylation, His-tag/immunofluorescence fusion forms) produced by recombinant methods.

The term "targeting BCMA", as used in the present invention, refers to the capability of specifically binding to BCMA (eg, human BCMA), for example, a chimeric antigen receptor targeting BCMA refers to a chimeric antigen receptor that can specifically bind to BCMA; for example, a scFv targeting BCMA refers to a single-chain antibody that can specifically bind to BCMA (eg, human BCMA).

The term "specific binding" or "specifically bind", as used in this disclosure, refers to a non-random binding reaction between two molecules, eg, a binding reaction between an antibody and an antigen. In certain embodiments, the antibody polypeptides provided by the present disclosure specifically binds human BCMA with a binding affinity (K_{D}) ≤10⁻⁶ M (for example, ≤5×10⁻⁷ M, ≤2×10⁻⁷ M, ≤10⁻⁷ M, ≤5×10⁻⁸ M, ≤2×10⁻⁸ M, ≤10⁻⁸ M, ≤5×10⁻⁹ M, ≤4×10⁻⁹M, ≤3×10⁻⁹M, ≤2×10⁻⁹ M or ≤10⁻⁹ M). K_{D} used in the present disclosure refers to the ratio of the dissociation rate to the binding rate (K_{off}/Kₒₙ), which can be determined by any conventional method known in the art, including but not limited to surface plasmon resonance method, microscale thermophoresis, high performance liquid chromatography-mass spectrometry, and flow cytometry (eg, FACS) method. In certain embodiments, K_{D} can be suitably determined by a flow cytometer.

The term "vector", as used herein, refers to a molecular tool for the transport, transduction and expression in a target cell of a contained exogenous gene of interest (eg, a polynucleotide of the present invention) that provides suitable nucleotide sequences for initiating transcription in the target cell, i.e. promoters. Vectors include isolated nucleic acids, and can be used to deliver isolated nucleic acids to the interior of cells. Numerous vectors are known in the art, including but not limited to linear polynucleotides, polynucleotides associated with ionic or amphiphilic compounds, plasmids, and viruses. Thus, the term "vector" includes autonomously replicating plasmids or viruses. The term should also be interpreted to include non-plasmid and non-viral compounds that facilitate transfer of nucleic acid into cells, e.g., polylysine compounds, liposomes, and the like. Examples of viral vectors include, but are not limited to, Sendai virus vectors, adenovirus vectors, adeno-associated virus vectors, retroviral vectors, lentiviral vectors, and the like.

An "expression vector", as used herein, refers to a vector comprising a recombinant polynucleotide that includes expression control sequences operably linked to the nucleotide sequence to be expressed. Expression vectors include sufficient cis-acting elements for expression; other elements for expression can be supplied by the host cell or in an *in vitro* expression system. Expression vectors include all those known in the art incorporating recombinant polynucleotides, such as plasmids (e.g., naked or contained in liposomes) and viruses (e.g., Sendai virus, lentivirus, retrovirus, adenovirus, and adeno-associated virus).

A "cloning vector", as used herein, refers to a DNA molecule such as a plasmid, cosmid, or phage that is capable of autonomous replication in a host cell. Cloning vectors typically contain one or a small number of restriction endonuclease recognition sites and marker genes, wherein exogenous DNA sequences are inserted in a defined manner at the restriction endonuclease recognition sites without losing essential biological function of the vector, the marker gene is suitable for the identification and selection of cells transformed with the cloning vector. Marker genes typically include genes that provide tetracycline resistance or ampicillin resistance.

Host cells can be any prokaryotic or eukaryotic cells that contains a cloning vector or expression vector, including those prokaryotic or eukaryotic cells that have been genetically engineered to contain the cloned gene in the chromosome or genome of the host cell. Suitable mammalian host cells include myeloma cells, such as SP2/0 cells and NSO cells, as well as Chinese hamster ovary (CHO) cells, hybridoma cell lines, and other mammalian host cells that can be used to express antibodies. Special transgenic animals with modified immune systems can also be used to make antibodies.

As used herein, "identity" refers to the sequence identity between two nucleic acid molecules or polypeptides. Identity can be determined by comparing the positions in each sequence aligned for comparison purpose. When a position in the compared sequences is occupied by the same base, then the molecules are identical at that position. The degree of similarity or identity between nucleic acid or amino acid sequences is a function of the number of identical or matching nucleotides at positions shared by the nucleic acid sequences. The identity between two sequences can be calculated by various alignment algorithms and/or programs, including those available as part of the GCG sequence analysis package (University of Wisconsin, Madison, Wis.), and FASTA or BLAST that can be used, for example, with default settings. For example, those skilled in the art can expect polypeptides that have at least 70%, 85%, 90%, 95%, 98%, or 99% identity to a particular polypeptide described herein and preferably a polypeptide that exhibits substantially the same function, and polynucleotides encoding the above-mentioned polypeptides.

As used herein, "isolated" means altered or removed from the natural state. For example, a nucleic acid or peptide naturally present in a living animal is not "isolated", but the same nucleic acid or peptide that is partially or completely separated from coexisting materials in its natural state is "isolated." An isolated nucleic acid or protein may be present in a substantially purified form, or may be present in a non-natural environment, such as in a host cell.

Unless otherwise specified, a nucleotide sequence "encoding" a nucleic acid molecule of a protein or an amino acid sequence of a protein, as used herein, includes a degeneracy of each other and all nucleotide sequences that encode the same amino acid sequence. The nucleotide sequence may also include one or more introns.

The term "variant", as used herein, means a variant of a polypeptide obtained by substituting at least one residue or adding at least one amino acid residue to the N- or C-terminus in the amino acid sequence of the parent molecule.

The term "subject", as used herein, includes any human or non-human animal. The term "non-human animal" includes all vertebrates such as mammals and non-mammals, for example, non-human primates, sheep, dogs, cats, horses, cows, chickens, rats, mice, amphibians, reptiles, and the like. Unless otherwise indicated, the terms "patient" or "subject" are used interchangeably. In the present invention, the preferred subject is a human.

The term "treating", as used herein, refers to administering to a subject an effective amount of cells having polynucleotide sequences of a target gene altered *ex vivo* according to the methods described herein, such that the subject has a reduction in at least one symptom of the disease or an improvement in the disease, for example, a beneficial or desired clinical outcome. For the purposes of the present disclosure, beneficial or desired clinical outcomes include, but are not limited to, reduction in one or more symptoms, reduction in disease severity, stabilization (ie, no worsening) of disease state, delay or slowing in disease progression, improvement or palliation of disease state, and remission (whether partial or complete remission), whether detectable or undetectable. Treatment may refer to prolonging survival as compared to expected survival if not receiving treatment. Thus, those skilled in the art realize that treatment can improve the disease condition, but may not be a complete cure of the disease. As used herein, the term "treatment" includes prevention. Alternatively, the treatment is "effective" if the progression of the disease is reduced or stopped. "Treatment" may also mean prolonging survival as compared to expected survival if not receiving treatment. Patients in need of treatment include those who have been diagnosed with disorders associated with the expression of polynucleotide sequences, and those who may develop such disorders due to genetic predisposition or other factors.

The term "autoimmune disease", as used herein, is defined as a disorder resulting from an autoimmune response. Autoimmune diseases are the result of inappropriate and excessive responses to self-antigens. Examples of autoimmune diseases include, but are not limited to, Addison's disease, alopecia areata, ankylosing spondylitis, autoimmune hepatitis, autoimmune mumps, Crohn's disease, diabetes (Type 1), Dystrophic epidermolysis bullosa, epididymitis, glomerulonephritis, Graves' disease, Guillain-Barré syndrome, Hashimoto's disease, hemolytic anemia, systemic lupus erythematosus, multiple sclerosis, myasthenia gravis, pemphigus vulgaris, psoriasis, rheumatic fever, rheumatoid arthritis, sarcoidosis, scleroderma, Sjogren's syndrome, spondyloarthropathy, thyroiditis, vasculitis, vitiligo, myxedema, malignant Anemia, ulcerative colitis, and the like.

Endpoints are included in cases where numerical limits or ranges are stated herein. In addition, all values and subranges within a numerical limit or range are specifically included.

### EXAMPLES

### Example 1. Synthesis of scFv Gene

In specific embodiments of the present invention, nucleic acids encoding scFv as set forth in Table 2 can be synthesized by genetic engineering method. The nucleic acid fragment encoding scFv of the present invention was synthesized at Beijing Biomed Gene Technology Co., Ltd. The scFv fragment contains a VH, a VL, and a linker region which links the VH domain and VL domain together to form an antigen binding site that specifically recognizes BCMA. A single-chain antibody is generally a sequence of one amino acid chain encoded by one nucleotide chain, the scFv may be further modified by amino acid deletions, insertions, substitutions, additions and other modifications known in the art. In addition, methods of introducing the above modifications in their nucleic acid sequences according to a certain amino acid sequence are also known to those skilled in the art, so that modification of scFv is preferably performed at the nucleic acid level.

### Example 2. Construction of CAR Molecules and Lentiviral Packaging

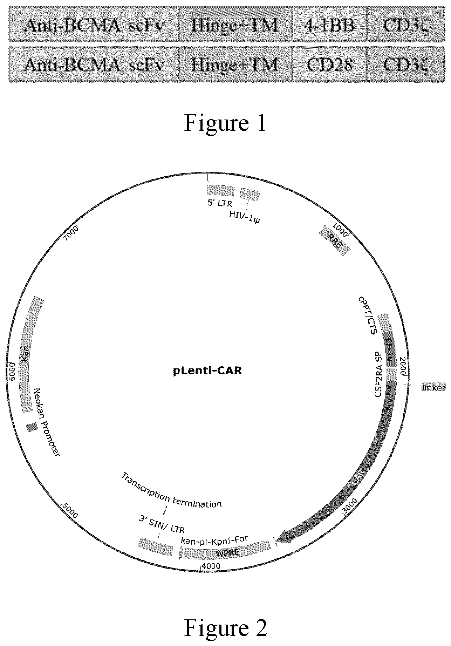

As shown in Figure 1, single-chain antibody scFv and hinge region, transmembrane structural domain, CD28 or 4-1BB costimulatory domain and CD3ζ domain together form the CAR structure in Example 1. The hinge region and transmembrane region are selected from CD8α.
1) The scFv gene targeting BCMA was synthesized by a gene synthesis method (Beijing Biomed Gene Technology Co., Ltd.). 2) Using the existing CAR plasmid as a template, the nucleic acid fragment containing CD8α hinge region, CD8α transmembrane region, intracellular region of 4-1BB (corresponding to NP_001552.2), and intracellular region of CD3ζ (corresponding to NP_000725.1) in a CAR molecule was cloned by PCR. 3) Using the gene obtained in step 1) and the nucleic acid fragment obtained in step 2) as templates, the complete nucleic acid fragment of CAR targeting BCMA was cloned by PCR. 4) The complete nucleic acid fragment obtained in step 3) was inserted into a lentiviral vector pLenti6.3/V5 (Thermo Fisher, Waltham, MA, USA) by restriction digestion and ligation to obtain a lentiviral transfer plasmid bearing the gene of CAR targeting BCMA (Figure 2). 5) The lentiviral packaging plasmids pLP/VSVG, pLP1/MDK, pLP2/RSK (Thermo Fisher, Waltham, MA, USA) and the transfer plasmid obtained in step 4) were transfected into HEK293T cells by Lipofectamine 3000 (Thermo Fisher, Waltham, MA, USA), and the medium was collected after 48 h. After removing cell debris by centrifugation at 300 g, the cells were centrifuged for 3 h at 25,000 rpm using an ultracentrifuge. The precipitate was dissolved in 1 mL of saline, which was the desired lentiviral vector.

### Example 3. Preparation of CAR-T cell

T cells were isolated from peripheral blood mononuclear cells (Miaotong (Shanghai) Biological Technology Co., Ltd., China) of healthy volunteers using CD3/CD28 Dynabeads (Thermo Fisher). The isolated and purified T cells (at this point the T cells were attached to CD3/CD28 Dynabeads) were cultured at 1.0×10⁶ cells/mL in X-VIVO 15 medium (Lonza, Switzerland) with IL-2 (Shandong Jintai Biological Engineering Co., Ltd., China) (500 IU/mL). After 48 h of cultivation, the T cells were infected with the above lentiviral vector. After 24 hours of virus infection, the cells were centrifuged and medium was changed, and fresh X-VIVO 15 containing IL-2 (500 IU/mL) was added to continue the culture. After 8 days of cell culture, all cells in the culture system were collected and Dynabeads were removed from the culture system using a magnetic rack, and T cells were centrifuged and counted.

### Example 4. Lentiviral Transduction Efficiency Assay

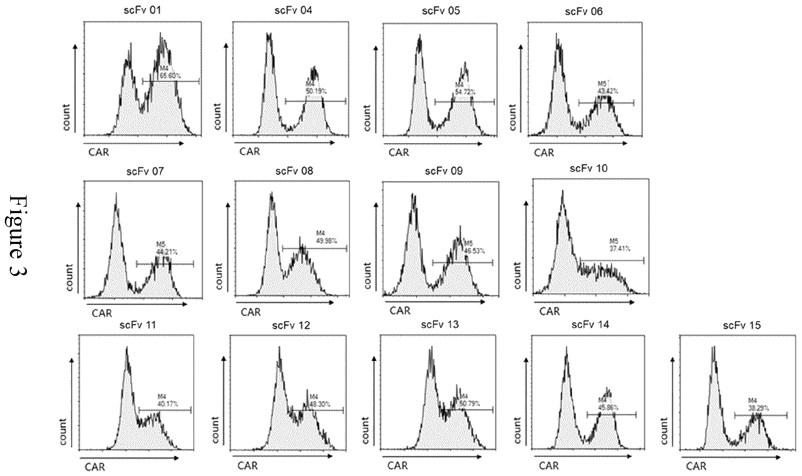

1.0×10⁶ cells were taken from the prepared CAR-T cells, the cells were resuspended with 200 µL of DPBS, and the supernatant was removed after centrifugation at 1000 g. The cell precipitate was resuspended with 100 µL of DPBS, 3 µL of antibody was added, incubated at room temperature for 20 min, and the cells were resuspended with 200 µL of DPBS, and the supernatant was removed after centrifugation at 1000 g. The cells were resuspended with 100 µL of DPBS, and the CAR content in each group of cells was detected by flow cytometer (NovoCyte 2060R, ACEA Biosciences, San Diego, CA, USA). As shown in Figure 3, the transduction efficiency of each CAR gene ranged from 35-65% at lentiviral infection MOI=0.5.

### Example 5. Detection of BCMA Expression on Surface of Target Cells

In this example, the expression of BCMA on the surface of human myeloma cell line NCI H929 and human leukemia cell line K562 was taken as an example.

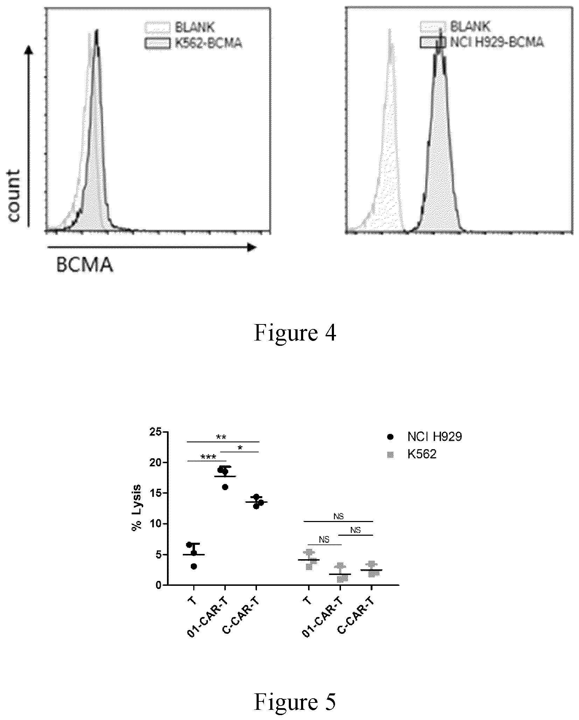

Both NCI H929 and K562 cells were purchased from ATCC. NCI H929 cells and K562 cells were cultured to the vigorous proliferation stage, and 1.0×10⁶ cells were taken from each. Cells were resuspended in 200 µL DPBS, centrifuged at 1000 g, and the supernatant was removed. The cell precipitate was resuspended in 100 µL DPBS, 5 µL fluorescence-labeled BCMA antibody (Miltenyi Biotec, USA) was added, incubated at room temperature for 20 minutes, the cells were resuspended in 200 µL DPBS, and the supernatant was removed after centrifugation at 1000 g. The cells were resuspended in 100 µL DPBS, and the ratio of BCMA expression in the two cells was detected by flow cytometer (NovoCyte 2060R, ACEA Biosciences, San Diego, CA, USA). As shown in Figure 4, the expression level of BCMA on surface of NCI H929 cells was high, while K562 cells hardly expressed BCMA.

### Example 6. Efficiency of BCMA CAR-T in Killing Target Cells

In this example, the detection of killing efficiency of CAR-Ts containing scFv that specifically recognizes BCMA, CD8α hinge region, CD8α transmembrane domain, 4-1BB costimulatory domain and CD3ζ domain on BCMA-positive target cells was taken as an example.

2×10⁶ NCI H929 target cells (ATCC) or BCMA expression-negative K562 cells was resuspended in 1 mL normal saline, 5 µL Calcein-AM (concentration 1 µg/µL, ThermoFisher, USA) was added, mixed gently, and then incubated in a 37°C water bath for 5 min to label target cells, washed twice with 10 mL of normal saline to remove excess dye, and cells were resuspended in 1 mL normal saline for counting. 1×10⁵ each of the above-labeled NCI H929 or K562 cells were added to each well of a 48-well cell culture plate (Corning Incorporated, Corning, NY, USA), various CAR-T cells were added according to E:T=5:1, the plate was placed in a 37°C, 5% CO₂ cell incubator for 6h, and the culture supernatant was taken for detection. The fluorescence values of the cell supernatants were detected with a fluorescence microplate reader (Varioscan Lux, ThermoFisher) (excitation wavelength: 495 nm, emission wavelength: 515 nm).

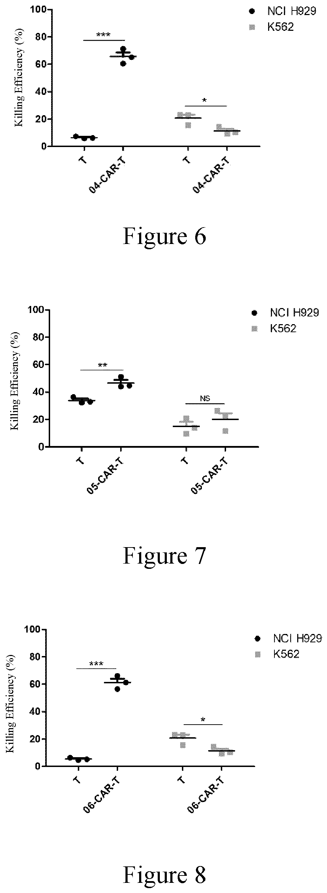

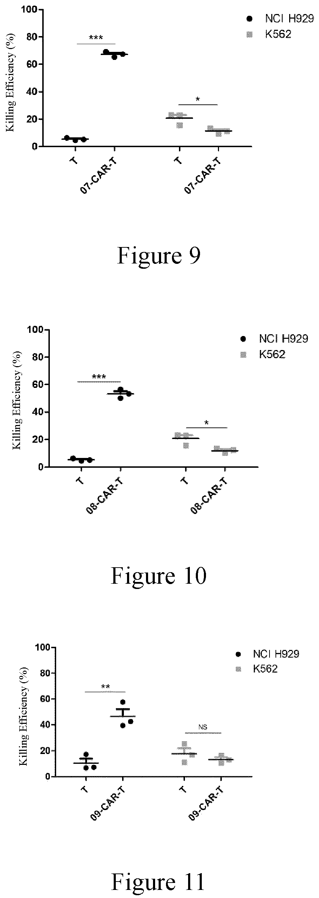

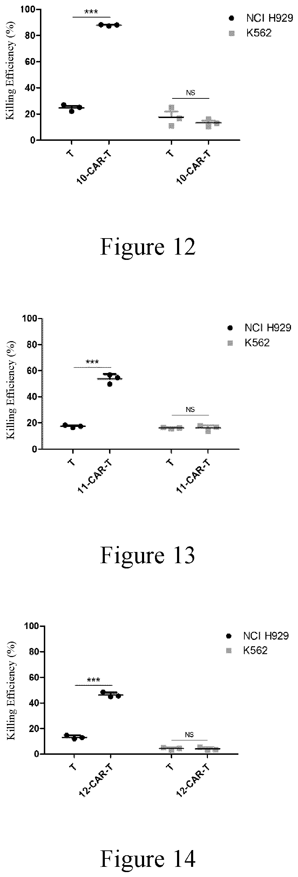

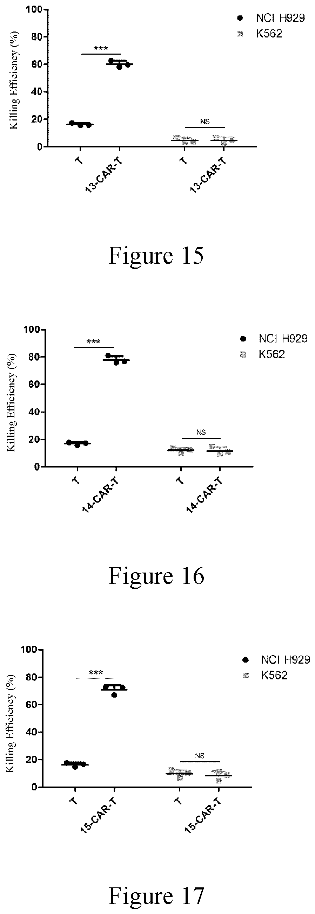

Figures 5 to 17 show that CAR-Ts with scFv targeting BCMA, co-stimulatory signaling domain and CD3ζ signaling domain can all effectively kill NCI H929 cells by recognizing BCMA target proteins, and are more effective than Bluebird Bio's BCMA-targeted CAR-Ts (C-CAR-T, sequence reference Seq No. 15 in patent WO2016/014789A2) on NCI H929 cells, but cannot effectively kill BCMA expression-negative K562 cells, indicating that these CAR-Ts can specifically recognize BCMA by scFv and activate CAR-Ts, cuasing CAR-Ts to kill tumor cells.

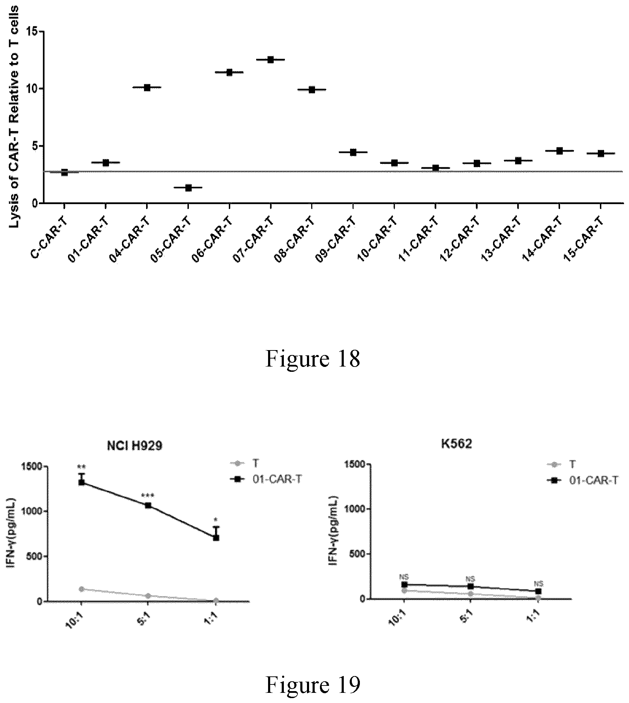

Figure 18 shows that compared with C-CAR-T, most CAR-T cells (except 05-CAR-T) have higher killing efficiency on NCI H929 cells, indicating that these CAR-T cells are more reactive on tumor cells.

### Example 7. Cytokine Expression of BCMA CAR-T Stimulated by Target Cells

In the present Example, the detection of the expression of the cytokine interferon gamma (IFN-γ) in BCMA-positive target cells (NCI H929) and BCMA negative cells (K562) after stimulation of CAR-Ts containing scFv-01 that specifically recognizes BCMA, CD8α hinge region, CD8α transmembrane domain, 4-1BB costimulatory domain and CD3ζ domain was taken as an example.

T cells and CAR-T cells were co-incubated with NCI H929 cells or K562 cells for 6 hours in a 37°C, 5% CO₂ incubator (the ratio of effector cells to target cells was 10:1, 5:1, 1:1, respectively), the supernatant was collected, and the CBA Human Th1/Th2/Th17 Cytokine Kit (BD Biosciences) was used to detect cytokine expression.

Figure 19 shows that, compared with T cells, BCMA CAR-T cells co-incubated with BCMA-positive target cells (NCI H929), IFN-γ-induced effect was significantly enhanced, and at the same time, with the increase of the ratio of effector cells to NCI H929 cells, cytokine release effect is enhanced. However, after co-incubation of BCMA CAR-T cells with BCMA-negative cells K562, IFN-γ-induced effect was not significantly different from that of the T cell group. The above experimental results show that the CAR-T cells of the present invention can specifically recognize the BCMA target antigen, thereby producing cytotoxicity-related cytokines.

## Claims

**1.** An isolated antibody that specifically binds to BCMA, comprising a heavy chain variable domain VH and a light chain variable domain VL,
the VH comprises a VH-CDR1 comprising SEQ ID NO: 1, a VH-CDR2 comprising SEQ ID NO: 2 and a VH-CDR3 comprising SEQ ID NO: 3;
the VL comprises a VL-CDR1 comprising SEQ ID NO: 4, a VL-CDR2 comprising SEQ ID NO: 5 and a VL-CDR3 comprising SEQ ID NO: 21.

**2.** The antibody of claim 1, wherein the VH-CDR1 comprises an amino acid sequence selected from the group consisting of SEQ ID NOs: 7, 8 and 9; the VH-CDR2 comprises an amino acid sequence selected from the group consisting of SEQ ID NOs: 10, 11, 12 and 13; the VH-CDR3 comprises an amino acid sequence selected from the group consisting of SEQ ID NOs: 14, 15 and 16; the VL-CDR1 comprises an amino acid sequence selected from the group consisting of SEQ ID NOs: 17, 18 and 19; the VL-CDR2 comprises the amino acid sequence of SEQ ID NO: 20; the VL-CDR3 comprises the amino acid sequence of SEQ ID NO: 21.

**3.** The antibody of claim 1, wherein the antibody comprising:
1) a VH comprising: a VH-CDR1 comprising SEQ ID NO: 7, a VH-CDR2 comprising SEQ ID NO: 10 and a VH-CDR3 comprising SEQ ID NO: 14; and a VL comprising: a VL-CDR1 comprising SEQ ID NO: 17, a VL-CDR2 comprising SEQ ID NO: 20 and a VL-CDR3 comprising SEQ ID NO: 21;
2) a VH comprising: a VH-CDR1 comprising SEQ ID NO: 8, a VH-CDR2 comprising SEQ ID NO: 11 and a VH-CDR3 comprising SEQ ID NO: 15; and a VL comprising: a VL-CDR1 comprising SEQ ID NO: 18, a VL-CDR2 comprising SEQ ID NO: 20 and a VL-CDR3 comprising SEQ ID NO: 21;
3) a VH comprising: a VH-CDR1 comprising SEQ ID NO: 8, a VH-CDR2 comprising SEQ ID NO: 12 and a VH-CDR3 comprising SEQ ID NO: 14; and a VL comprising: a VL-CDR1 comprising SEQ ID NO: 17, a VL-CDR2 comprising SEQ ID NO: 20 and a VL-CDR3 comprising SEQ ID NO: 21;
4) a VH comprising: a VH-CDR1 comprising SEQ ID NO: 9, a VH-CDR2 comprising SEQ ID NO: 13 and a VH-CDR3 comprising SEQ ID NO: 16; and a VL comprising: a VL-CDR1 comprising SEQ ID NO: 19, a VL-CDR2 comprising SEQ ID NO: 20 and a VL-CDR3 comprising SEQ ID NO: 21.

**4.** The antibody of claim 1, wherenin the VH comprises an amino acid sequence selected from the group consisting of SEQ ID NOs: 23, 24, 25, 26, 27, 28, 29, 30, 31 and 32, or comprises an amino acid sequence with at least 85%, preferably at least 90%, more preferably at least 95%, further preferably at least 99% identity thereto; the VL comprises an amino acid sequence selected from the group consisting of SEQ ID NOs: 33, 35, 36, 37, 38, 39, 40, 41, 42 and 43, or comprises an amino acid sequence with at least 85%, preferably at least 90%, more preferably at least 95%, further preferably at least 99% identity thereto.

**5.** The antibody of claim 1, comprising:
1) a VH comprising SEQ ID NO: 23 or an amino acid sequence with at least 85%, preferably at least 90%, more preferably at least 95%, further preferably at least 99% identity thereto; and a VL comprising SEQ ID NO: 33 or an amino acid sequence with at least 85%, preferably at least 90%, more preferably at least 95%, further preferably at least 99% identity thereto;
2) a VH comprising SEQ ID NO: 24 or an amino acid sequence with at least 85%, preferably at least 90%, more preferably at least 95%, further preferably at least 99% identity thereto; and a VL comprising SEQ ID NO: 33 or an amino acid sequence with at least 85%, preferably at least 90%, more preferably at least 95%, further preferably at least 99% identity thereto;
3) a VH comprising SEQ ID NO: 25 or an amino acid sequence with at least 85%, preferably at least 90%, more preferably at least 95%, further preferably at least 99% identity thereto; and a VL comprising SEQ ID NO: 35 or an amino acid sequence with at least 85%, preferably at least 90%, more preferably at least 95%, further preferably at least 99% identity thereto;
4) a VH comprising SEQ ID NO: 26 or an amino acid sequence with at least 85%, preferably at least 90%, more preferably at least 95%, further preferably at least 99% identity thereto; and a VL comprising SEQ ID NO: 36 or an amino acid sequence with at least 85%, preferably at least 90%, more preferably at least 95%, further preferably at least 99% identity thereto;
5) a VH comprising SEQ ID NO: 27 or an amino acid sequence with at least 85%, preferably at least 90%, more preferably at least 95%, further preferably at least 99% identity thereto; and a VL comprising SEQ ID NO: 37 or an amino acid sequence with at least 85%, preferably at least 90%, more preferably at least 95%, further preferably at least 99% identity thereto;
6) a VH comprising SEQ ID NO: 28 or an amino acid sequence with at least 85%, preferably at least 90%, more preferably at least 95%, further preferably at least 99% identity thereto; and a VL comprising SEQ ID NO: 38 or an amino acid sequence with at least 85%, preferably at least 90%, more preferably at least 95%, further preferably at least 99% identity thereto;
7) a VH comprising SEQ ID NO: 29 or an amino acid sequence with at least 85%, preferably at least 90%, more preferably at least 95%, further preferably at least 99% identity thereto; and a VL comprising SEQ ID NO: 33 or an amino acid sequence with at least 85%, preferably at least 90%, more preferably at least 95%, further preferably at least 99% identity thereto;
8) a VH comprising SEQ ID NO: 30 or an amino acid sequence with at least 85%, preferably at least 90%, more preferably at least 95%, further preferably at least 99% identity thereto; and a VL comprising SEQ ID NO: 39 or an amino acid sequence with at least 85%, preferably at least 90%, more preferably at least 95%, further preferably at least 99% identity thereto;
9) a VH comprising SEQ ID NO: 31 or an amino acid sequence with at least 85%, preferably at least 90%, more preferably at least 95%, further preferably at least 99% identity thereto; and a VL comprising SEQ ID NO: 40 or an amino acid sequence with at least 85%, preferably at least 90%, more preferably at least 95%, further preferably at least 99% identity thereto;
10) a VH comprising SEQ ID NO: 32 or an amino acid sequence with at least 85%, preferably at least 90%, more preferably at least 95%, further preferably at least 99% identity thereto; and a VL comprising SEQ ID NO: 41 or an amino acid sequence with at least 85%, preferably at least 90%, more preferably at least 95%, further preferably at least 99% identity thereto;
11) a VH comprising SEQ ID NO: 28 or an amino acid sequence with at least 85%, preferably at least 90%, more preferably at least 95%, further preferably at least 99% identity thereto; and a VL comprising SEQ ID NO: 42 oran amino acid sequence with at least 85%, preferably at least 90%, more preferably at least 95%, further preferably at least 99% identity thereto; or
12) a VH comprising SEQ ID NO: 29 or an amino acid sequence with at least 85%, preferably at least 90%, more preferably at least 95%, further preferably at least 99% identity thereto; and a VL comprising SEQ ID NO: 43 or an amino acid sequence with at least 85%, preferably at least 90%, more preferably at least 95%, further preferably at least 99% identity thereto.

**6.** The antibody of claim 1, comprising an amino acid sequence as set forth in SEQ ID NOs: 44, 45, 47, 48, 49, 50, 51, 52, 53, 54, 55 or 56, or an amino acid sequence with at least 85%, preferably at least 90%, more preferably at least 95%, further preferably at least 99% identity thereto.

**7.** The antibody of any one of claims 1-6, wherein the antibody is scFv.

**8.** A chimeric antigen receptor targeting BCMA, comprising the scFv of claim 7, optionally further comprising at least one of hinge region, transmembrane region, costimulatory domain, and intracellular signal transduction domain.

**9.** The chimeric antigen receptor of claim 8, wherein the costimulatory domain is selected from CD27, CD28, 4-1BB, OX-40, CD30, CD40, PD-1, ICOS, LFA-1, CD2, CD7, LIGHT, NKG2C, B7-H3, or any combination thereof; preferably, the costimulatory domain is selected from 4-1BB, the 4-1BB comprises the amino acid sequence of SEQ ID NO: 57; preferably, the intracellular signal transduction domain is selected from CD3ζ, comprising the amino acid sequence of SEQ ID NO: 58.

**10.** An isolated nucleic acid molecule encoding the antibody of any one of claims 1-7 or the chimeric antigen receptor of any one of claims 8-9.

**11.** A vector comprising the nucleic acid molecule of claim 10.

**12.** A host cell comprising the nucleic acid molecule of claim 10 or the vector of claim 11.

**13.** A composition or kit comprising at least one of the antibody of any one of claims 1-7, the chimeric antigen receptor of claim 8 or 9, the nucleic acid molecule of claim 10, the vector of claim 11, the host cell of claim 12, as well as pharmaceutically acceptable excipient(s), diluent(s) or carrier(s).

**13.** Use of the antibody of any one of claims 1-7, the chimeric antigen receptor of claim 7 or 8, the nucleic acid molecule of claim 9, the vector of claim 10, the host cell of claim 11, or the composition of claim 12, in preparation of a medicament for the prevention or treatment of B-cell associated neoplastic diseases, autoimmune diseases, infectious diseases caused by viruses or bacteria.

**14.** The use of claim 13, wherein the B-cell associated neoplastic disease is at least one selected from: acute leukemias such as acute lymphoblastic leukemia, acute myelocytic leukemia, acute myelogenous leukemia and myeloblastic leukemia, promyelocytic leukaemia, myelomonocytic leukaemia, monocytic leukaemia and erythroleukemia; chronic leukemias such as chronic myelocytic (granulocytic) leukemia, chronic myelogenous leukemia, and chronic lymphocytic leukemia; polycythemia vera, lymphoma, mantle cell lymphoma, diffuse large B-cell lymphoma, Hodgkin's disease, non-Hodgkin's lymphoma, multiple myeloma, Waldenstrom's macroglobulinemia, heavy chain disease, myelodysplastic syndrome, hairy cell leukemia, and myelodysplasia.
